Europäisches Patentamt

European Patent Office

Office européen des brevets

(19)

(11) Veröffentlichungsnummer: **0 432 429 A1**

## (12) EUROPÄISCHE PATENTANMELDUNG

(21) Anmeldenummer: 90120766.2

(22) Anmeldetag: 30.10.90

(51) Int. Cl.5: **B01J 8/06**, C07C 29/15

(30) Priorität: 15.12.89 DE 3941407

(43) Veröffentlichungstag der Anmeldung:
19.06.91 Patentblatt 91/25

(84) Benannte Vertragsstaaten:
CH DE DK GB IT LI

(71) Anmelder: Uhde GmbH
Friedrich-Uhde-Strasse 15 Postfach 262
W-4600 Dortmund 1(DE)

(72) Erfinder: Bähnisch, Hans-Joachim
Wembersweg 30
W-4600 Dortmund 76(DE)

(74) Vertreter: Patentanwälte Meinke und
Dabringhaus Dipl.-Ing. J. Meinke Dipl.-Ing. W.
Dabringhaus
Westenhellweg 67
W-4600 Dortmund 1(DE)

(54) **Rohrreaktor.**

(57) Bei einem Reaktor (R) mit körniges Katalysatormaterial enthaltenden Rohren (4,17), die mit ihrem oberen Ende in einem oberen Rohrboden (5) und mit ihrem unteren Ende in einem unteren Rohrboden (6) angeordnet und in Betrieb von wenigstens den zwischen den Rohrböden gebildeten Raum durchströmenden Kühlmittel umgeben sowie von einem umzusetzenden Gasgemisch durchströmt sind, wobei einer der Rohrböden fest am Reaktormantel (7) angeordnet und der andere Rohrboden ohne unmittelbare Verbindung relativ zum Reaktormantel (7) bewegbar ausgebildet ist.

Wird der bewegbare Rohrboden (5) mit einem eigenen Kugelboden bzw. Gasdom (3) ausgebildet und zwischen diesem Gasdom (3) und Reaktormantel (7) ein oder mehrere wenigstens bereichsweise flexible Kompensationsgasrohre (2) mit einer festen Verbindung zum Reaktormantel (7) vorgesehen.

EP 0 432 429 A1

## "ROHRREAKTOR"

Die Erfindung richtet sich auf einen Reaktor mit körniges Katalysatormaterial enthaltenden Rohren, die mit ihrem oberen Ende in einem oberen Rohrboden und mit ihrem unteren Ende in einem unteren Rohrboden angeordnet und im Betrieb von wenigstens den zwischen den Rohrböden gebildeten Raum durchströmenden Kühlmittel umgeben sowie von einem umzusetzenden Gasgemisch durchströmt sind.

Derartige Rohrreaktoren sind in vielfältigen Ausgestaltungen bekannt. Gemeinsam ist den bekannten Lösungen jedoch, daß die Rohrböden jeweils mit dem Reaktormantel verschweißt sind, um eine ausreichende mechanische Stabilität zwischen dem Reaktormantel und den mit Katalysator gefüllten Rohren zu gewährleisten. Bei Einsatz solcher Reaktoren für exotherme Reaktionen können nun innerhalb der Katalysatorrohre und der Rohrböden erhebliche Temperaturunterschiede auftreten, die zu einer entsprechenden Materialbelastung insbesondere durch eine Dehnung der Bauteile führen können, was sich auch nicht durch eine Kühlmittelbeaufschlagung vollständig beseitigen läßt. Es sind deshalb unterschiedliche Lösungen bekannt, mit denen diesen Nachteilen zu begegnen versucht wird.

So zeigt die DE-OS 36 01 366 einen Reaktor, bei dem über dem oberen Rohrboden ein senkrecht bewegbarer Verteilerboden angeordnet ist, der Röhrchen aufweist, welche in den oberen Bereich der Rohre hineinragen. Mit dieser konstruktiv sehr aufwendigen Ausbildung soll erreicht werden, daß die Rohre im Bereich des oberen Rohrbodens und unmittelbar darunter vom Katalysator freibleiben, derart, daß in diesen Bereichen noch keine Reaktionen und damit keine Wärmeentwicklung stattfindet. Eine andere Lösung zeigt die DE-AS 21 53 437, bei der aufgrund einer speziellen Materialauswahl der Rohre und des Mantels die Wärmeausdehnung der Materialien in erlaubten Grenzen gehalten werden soll. Diese insbesondere austenitisch-ferritischen Stähle sind jedoch sehr aufwendig, so daß der Einsatz derartiger Reaktoren unwirtschaftlich ist.

Aus der GB-PS 367 772 ist bereits ein zylinderförmiger Röhrenreaktor für die Ammoniak- und Methanolsynthese bekannt, wobei dort der obere Rohrboden im Reaktorgehäuse festgelegt ist, während der untere Rohrboden zur Längenausdehnung beweglich ist mit einem zylindrischen, eine axiale Bewegung relativer Durchtrittsöffnung in der Behälterwand ermöglichenden Rohr innerhalb vergleichsweise aufwendiger Stopfbuchsen. Aus der DE-39 22 446-A1 ist ein zylinderförmiger Röhrenreaktor für eine Dampfreformierung, d.h. eine Kohlenwasserstoffspaltung, bekannt mit einer endothermen, d.h. nicht exothermen Reaktion, wobei dort wiederum eine Konstruktion gewählt ist mit einer oberen festen Rohrplatte mit hängenden Rohren mit einer unteren Rohrplatte an einem weiteren Aufnahmegehäuse, das wiederum über ein Einströmrohr jeweils an der Gehäusewand festgelegt ist.

Aufgabe der Erfindung ist die Schaffung einer Lösung, die auf konstruktiv möglichst einfache Weise einen Ausgleich der durch die Temperaturunterschiede auftretenden Dehnungen der Bauteile eines derartigen Reaktors ermöglicht.

Diese Aufgabe wird mit einem Reaktor der eingangs bezeichneten Art erfindungsgemäß durch die kennzeichnenden Merkmale des Anspruches 1 gelöst.

Mit dieser Ausbildung ist es auf überraschend einfache Weise möglich, durch Temperaturunterschiede im Reaktor auftretende Bauteildehnungen, insbesondere der Katalysatorrohre und der Rohrböden auszugleichen, ohne daß dadurch die mechanische Stabilität des Reaktors beeinträchtigt wird. Das flexible Kompensationsgasrohr nimmt dabei entsprechend die Bauteilausdehnungsschwankungen auf, so daß keine Bauteilspannungen aufgrund behinderter Wärmedehnungen auftreten können. Aufwendige Dichtprobleme schaffende Stopfbuchsenlösungen od. dgl. in der Reaktorwand sind daher entbehrlich.

Die mechanische Stabilität des Reaktors wird durch die erfindungsgemäße Ausbildung des bewegbaren Rohrbodens mit einem eigenen Kugelboden besonders vorteilhaft sichergestellt. Reaktoren der eingangs bezeichneten Art werden im allgemeinen mit einem Gasgemischdruck betrieben, der entweder über oder unter dem Kühlmitteldruck liegt. Für eine derartige Differenzdruckbeanspruchung, die je nach Betriebsweise Werte bis zu 130 bar annehmen kann, kann ein Kugelboden mit dünnerer Wandstärke als andere Bodenformen, wie z.B. ebene oder gewölbte Böden ausgeführt werden, wodurch Gewicht gespart wird. Überraschenderweise stellte sich heraus, daß im Betrieb des Reaktors die Bauteiltemperatur des Kugelbodens den Wert der Kühlmitteltemperatur annimmt, während die Bauteiltemperatur des oberen Rohrbodens etwa den Wert der Gasgemischtemperatur annimmt, was unterschiedliche Dehnungen der beiden Bauteile in radialer Richtung zur Folge hat und daß der vergleichsweise dünne Kugelboden diese Dehnungsdifferenz ohne Materialüberlastung aufzunehmen vermag. Die erfindungsgemäße Kombination des oberen Rohrbodens mit einem Kugelboden erspart auf einfache Weise Kompensationsmaßnahmen an dieser Stelle.

Weitere vorteilhafte Ausgestaltungen der Erfindung ergeben sich aus den Unteransprüchen.

Dabei ist es vorteilhaft, den bewegbaren Rohrboden mit einem eigenen Gasdom auszustatten, wobei zwischen Gasdom einerseits und festem Reaktormantel andererseits wenigstens das oder die Kompensationsrohre vorgesehen sind.

Die Gestaltung und Anordnung der Kompensationsrohre kann dabei unterschiedlich vorgenommen werden. Entweder bei stehender Bauweise und sich oben befindlichem bewegbaren Rohrboden direkt von oben oder aber bei gleicher Anordnung des Rohrbodens von unten, wobei dann das Kompensationsrohr z.B. zentrisch dem Bereich der Katalysatorrohre durchsetzt.

Vorteilhaft ist auch, wenn die dem Gasdom zugeordneten Einströmbereiche des oder der Kompensationsrohre mit Gasverteilereinrichtungen ausgerüstet sind, um hier eine optimale Beaufschlagung der Katalysatorrohre zu erreichen. Vorteilhaft kann dabei auch in weiterer Ausgestaltung sein, wenn zwischen Außenfläche des Gasdomes und der Innenfläche des Reaktormantels ein Kühlmitteldampfdom ausgebildet ist. Auch dies führt zu einer optimalen Prozeßführung. Diesem Ziel dient auch ein Tröpfchenabscheider im Bereich des Kühlmitteldampfdomes.

Es ist besonders zweckmäßig, wenn wenigstens am Austritt eines der körniges Katalysatormaterial enthaltenden Rohre eine Gasdurchflußregeleinrichtung vorgesehen ist, wobei bevorzugt dieses Rohr auch einen größeren Querschnitt als die übrigen Rohre aufweist. Auf diese Weise können die Reaktionsbedingungen im Reaktor entsprechend überwacht und gesteuert werden. Dabei kann dieses Rohr auch ohne Katalysatorfüllung ausgestattet sein.

Der erfindungsgemäße Reaktor ist insbesondere zur Herstellung von Methanol aus einem Wasserstoff, Kohlenmonoxid und Kohlendioxid enthaltenden Synthesegas geeignet.

Die Erfindung ist nachstehend anhand der Zeichnung beispielsweise näher erläutert. Diese zeigt in

Fig. 1 bis 5 einen vereinfachten Schnitt durch einen Reaktor gemäß der Erfindung in unterschiedlichen Gestaltungsvarianten sowie in

Fig. 6 ein Verfahrensfließbild einer Methanolsynthese unter Einsatz erfindungsgemäßer Reaktoren.

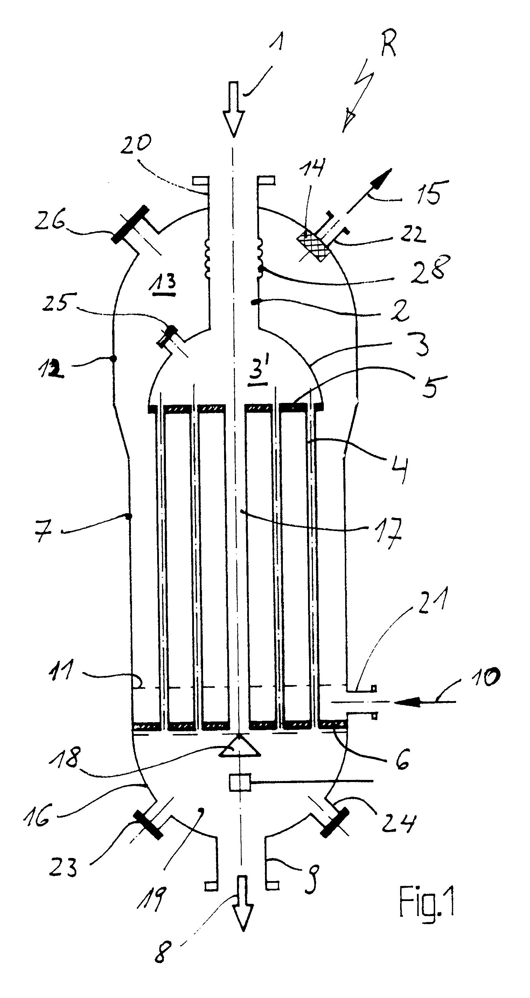

Ein Reaktor zur Durchführung katalytischer Gasreaktionen, insbesondere für die Methanolsynthese, ist in der Zeichnung gemäß Fig. 1 allgemein mit R bezeichnet. Der Reaktor R weist zunächst einen im wesentlichen zylindrischen, druckfesten Reaktormantel 7 auf, an dessen Oberseite ein oberer Kugelboden bzw. Gasdom 12 und an dessen Unterseite ein unterer Kugelboden bzw. Gasdom 16 angeordnet ist.

Im unteren Bereich des zylindrischen Reaktormantels 7 ist ein unterer Rohrboden 6 umlaufend angeschweißt, an dem seinerseits eine Mehrzahl von senkrecht stehend angeordneten Rohren 4 mit körniger Katalysatorfüllung angeschweißt sind. Den oberen Abschluß dieser Rohre 4 bildet ein oberer Rohrboden 5, der, wie der untere Rohrboden 6, im Bereich der Rohranschlüsse jeweils entsprechende Öffnungen aufweist, wobei der untere Rohrboden 6 in bekannter Weise Einrichtungen aufweist, die ein Herausrutschen des Katalysators verhindern, jedoch den Gasdurchtritt ermöglichen.

Wesentlich für die Erfindung ist nun, daß der obere Rohrboden 5 nicht in Verbindung mit dem Reaktormantel 7 steht, d.h. insbesondere nicht an diesem befestigt ist. Vielmehr ist am oberen Rohrboden 5 ein halbkugelförmiger Gasdom 3 druckdicht angeordnet, an dessen Oberseite ein senkrecht angeordnetes, in Längsrichtung bereichsweise flexibles Kompensationsgasrohr 2. z.B. durch Verschweißen druckdicht befestigt ist. Dieses Kompensationsrohr 2 ist durch den Reaktormantel des oberen Kugelbodens 12 hindurchgeführt und gleichzeitig fest an diesem angeordnet, wobei der äußere Bereich des Kompensationsrohres 2 starr ausgebildet und nachfolgend als Synthesegaseintrittsstutzen 20 bezeichnet ist.

Synthesegas wird in Richtung des Pfeiles 1 durch den Synthesegaseintrittsstutzen 20 hindurch in das Kompensationsgasrohr geleitet und tritt von diesen in die Gasvorkammer 31 ein, wo es sich gleichmäßig verteilt und über den oberen Rohrboden 5 in die Katalysatorrohre 4 gelangt. In diesen Katalysatorrohren findet die eigentliche Reaktion statt, was mit einer Wärmeentwicklung verbunden ist. Treten nun aufgrund der Wärmeentwicklung Dehnungen der Rohre 4 oder auch des Rohrbodens 5 auf, so werden diese Dehnungen vom Kompensationsgasrohr 2 aufgenommen, das sich in Längsrichtung aufgrund seiner Flexibilität zusammenziehen kann.

Das entstandene Produktgas tritt durch den unteren Rohrboden 6 in einen durch diesen und den unteren Kugelboden 16 begrenzten Produktgassammelraum 19 ein, aus dem es durch ein Produktgasaustrittsstutzen 9 in Richtung des Pfeiles 8 austreten kann. Wie in Fig. 1 erkennbar, ist das mittig angeordnete Katalysatorrohr gegenüber den übrigen Katalysatorrohren 4 mit vergrößertem Querschnitt ausgebildet, dieses zentrale Rohr ist mit 17 bezeichnet. Am Austritt dieses Rohres 17 ist eine Gasdurchflußregeleinrichtung 18 vorgesehen, über die die Synthesegasmenge entsprechend geregelt werden kann.

Zur Kühlung des Reaktors ist ein Kühlsystem vorgesehen, das zunächst mehrere Kühlmitteleintrittsstutzen 21 aufweist, die im unteren Bereich des zylindrischen Reaktormantels 7 oberhalb des unteren Rohrbodens 6 angeordnet sind. Durch diese Stutzen 21 tritt Kühlmittel in Richtung der Pfeile 10 in den Reaktor ein und wird aufgrund einer in diesem Bereich angeordneten perforierten Verteilerplatte 11 gleichmäßig auf den zwischen den beiden Rohrböden 5 und 6 gebildeten Raum verteilt, d.h. es sorgt für eine Kühlung der Reaktorrohre 4.

Entstehender Kühlmitteldampf kann zwischen dem oberen Rohrboden 5 und dem zugeordneten Reaktormantel in den Bereich des oberen Kugelbodens 12 eintreten, der zwischen sich und der Gasvorkammer 3' sowie dem Gaskompensationsrohr 2 einen Kühlmitteldampfdom 13 bildet. Der entstandene Kühlmitteldampf kann über einen Kühlmitteldampfaustrittsstutzen 22 mit Tröpfchenabscheider 14 in Richtung des Pfeiles 15 abgezogen werden.

Weiterhin sind zur Beschickung des Reaktors R noch zusätzliche Einrichtungen vorgesehen, nämlich ein Katalysatoreinfüllstutzen 25 im Bereich der Gasvorkammer 3' sowie ein oder mehrere Katalysatorauslaßstutzen 23 im Bereich des unteren Kugelbodens 19. Ferner sind noch Mannlöcher 24 und 26 angedeutet.

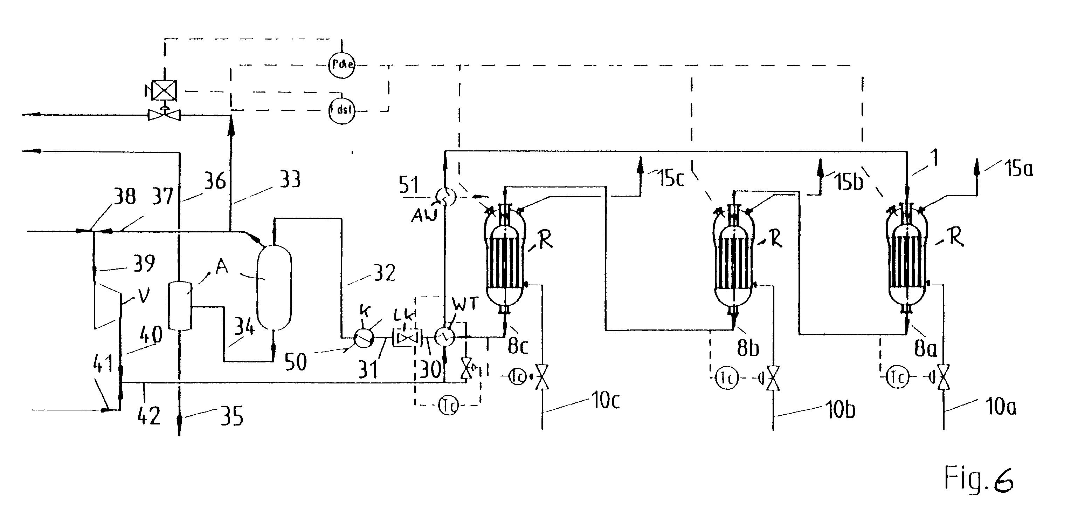

In Fig. 6 ist die Anwendung eines Reaktors nach Fig. 1 dargestellt, wobei Fig. 6 eine Methanolsyntheseanlage zeigt, in der drei Reaktoren R eingesetzt sind, selbstverständlich könnte hier aber auch eine andere Anzahl vorgesehen sein.

Frischgas 38 und Kreislaufgas 37 werden zunächst zu einem Gasstromm 39 gemischt, welcher anschließend in einem Ver dichter V zu einem Gasstrom 40 verdichtet wird. Dieser verdichtete Gasstrom 40 wird dann mit einem $CO_2$-Gasstrom 41 zu einem Gasstrom 42 gemischt.

Der Gasstrom 42 wird daraufhin in einem Gas-/Gaswärmeaustauscher WT bzw. in der Anfahrphase in einem Anfahrwärmeaustauscher AW, der mit einem externen Heizmedium 51 beaufschlagt ist, vorgewärmt. Der vorgewärmte Synthesegasstrom wird dann in Richtung des Pfeils 1 in den ersten Reaktor R eingeleitet, wobei nach Reaktion in demselben das austretende Gas über den Produktgasaustritt 8a abgeführt wird. Zur Kühlung des Reaktors R wird durch den Kühlmitteleintritt 10a Kühlmittel in den Reaktor eingeleitet, das als Kühlmitteldampf aus dem Dampfaustritt 15a abgezogen wird.

Das Produktgas aus dem ersten Reaktor R wird anschließend in den zweiten Reaktor R eingeleitet und nach Reaktion in diesem durch die Leitung 8b abgezogen. Die Kühlung des zweiten Reaktors R ist durch die Zuleitung 10b und die Dampfaustrittsleitung 15b angedeutet.

Das Methanol-Produktgas aus dem zweiten Reaktor R wird anschließend weiteren Reaktoren zugeführt, je nach den gewünschten Reaktionsabläufen, letztendlich gelangt es jedoch in einen letzten Reaktor R, aus dem es durch die Leitung 8c austritt. Die Kühlmittelzufuhr ist hier mit 10c, der Kühlmitteldampfabzug mit 15c bezeichnet.

Das aus dem letzten Reaktor R austretende Produktgas wird dann zur Abkühlung durch den Gas-/Gaswärmeaustauscher WT geleitet. Das abgekühlte Produktgas 30 kann dann in einem Luftkühler LK weiter abgekühlt werden, dieser abgekühlte Gasstrom ist mit 31 bezeichnet. Eine nochmalige Kühlung kann in einem nachgeschalteten Kondensator K erfolgen, wobei anschließend das abgekühlte Produktgas 32 vorzugsweise in einem zweigeteilten Abscheider A in Rohmethanol 35, Kreislaufgas 37, Entspannungsgas 36 und Purgegas 33 getrennt wird.

Die einzelnen Reaktoren werden bevorzugt mittels einer Druckdifferenzregelung geschützt, wobei die führende Größe der Dampfdruck ist. Der Dampfdruck beträgt etwa maximal 40 bar und eine einstellbare Druckdifferenz kann z.B. zwischen 10 und 60 bar liegen, der Synthesedruck ist kapazitätsabhängig, so daß die Reaktoreinbauten entsprechend nur der Druckdifferenz auszulegen sind.

In den Fig. 1 bis 5 sind unterschiedliche Varianten einer Reaktorausbildung und der Anordnung des Kompensationsrohres dargestellt, wobei dort bei funktionsmäßig gleichen Bauteilen die gleichen Bezugszeichen wie bei der Gestaltungsvariante nach Fig. 1 gewählt wurden ggf. durch die Hinzufügung von Kleinbuchstaben, wie "a", "b", "c" ... .

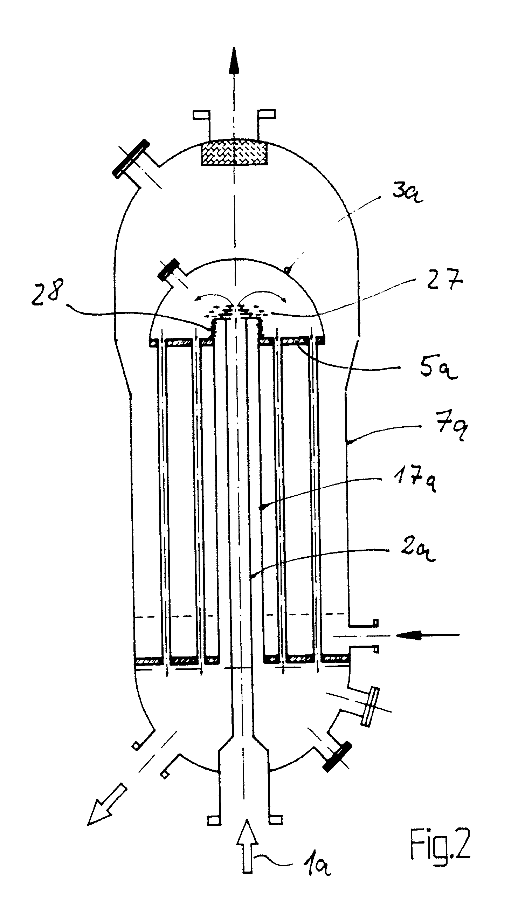

Im Ausführungsbeispiel gemäß Fig. 2 wird das zentrische Katalysatorrohr 17a von unten vom Kompensationsgasrohr 2a durchsetzt. Dieses weist an seiner dem inneren Kugelboden bzw. Gasdom 3a zugeordneten freien Ende eine Gasverteilereinrichtung 27 auf, der die Ausdehnung kompensierende Balgenbereich ist in Fig. 2 mit 28 bezeichnet.

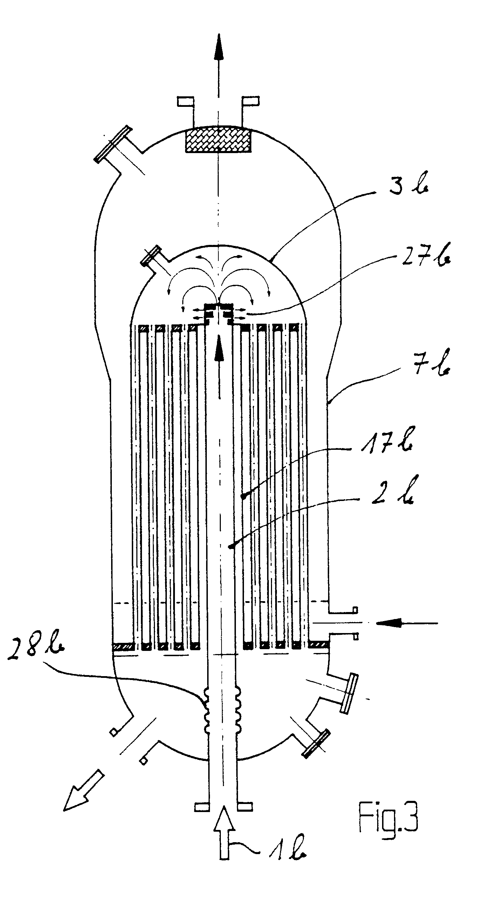

In Fig. 3 ist eine ähnliche Lösung dargestellt, dort durchsetzt das Kompensationsgasrohr 2b wiederum das zentrische Katalysatorrohr 17b von unten nach oben, auch dort ist eine obere Gasverteileinrichtung 27b vorgesehen, die Balgenkompensationsstrecke ist dort allerdings im unteren Bereich vorgesehen und mit 28b bezeichnet.

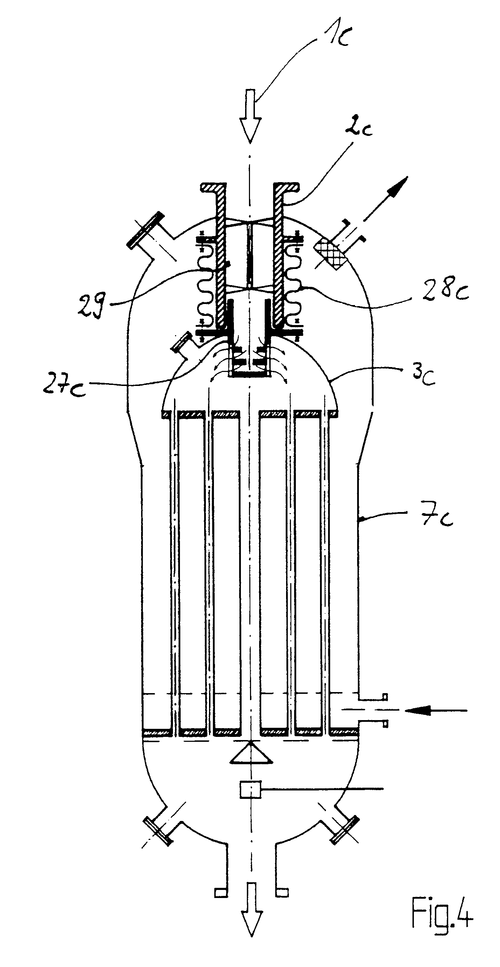

Fig. 4 zeigt eine Beaufschlagung durch ein Kompensationsgasrohr 2c von oben. Hier ist eine Faltenbalgstrecke 28c vorgesehen, die sich innen an einem Stützrohrbereich 29 abstützt, wobei unten im unteren Bereich, dem Kugelboden bzw. Gasdom 3c zugeordnet, ein Gleitrohrstutzen eines Verteilers 27c vorgesehen ist.

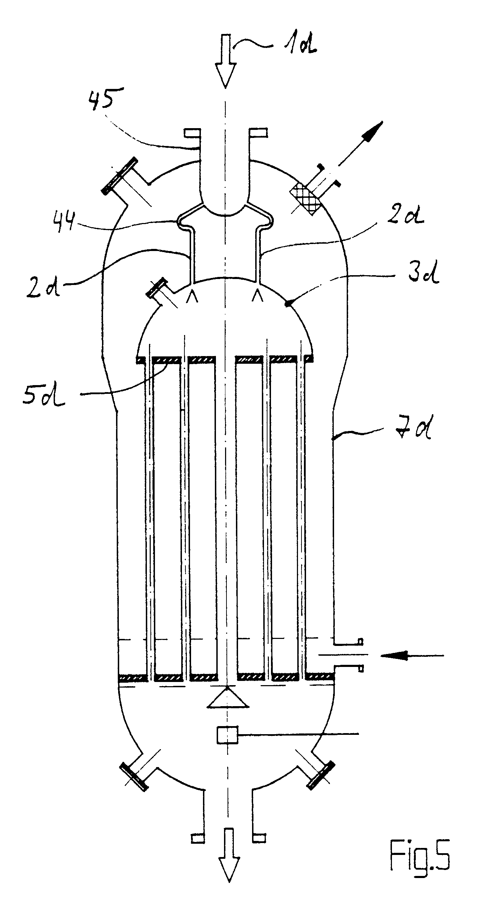

Schließlich zeigt Fig. 5 eine Beaufschlagung des oberen Kugelbodens bzw. Gasdomes 3d mit einer Vielzahl von Kompensationsrohren 2d, an denen aus Darstellungsgründen in Fig. 5 nur zwei wiedergegeben sind. Diese Vielzahl von Kompensationsgasrohren 2d ist jeweils mit einem Dehnungsbogen, allgemein mit 44 bezeichnet, ausgerüstet, sie finden ihren Anfang in einer Art Verteilerrohr 45 und enden, wie bereits beschrieben, im inneren Kugelboden bzw. Gasdom 3d, der dem bewegbaren oberen Rohrboden 5d zugeordnet ist.

Natürlich ist die Erfindung nicht auf die in der Zeichnung dargestellten Ausführungsbeispiele beschränkt. Weitere Ausgestaltungen der Erfindung sind möglich, ohne den Grundgedanken zu verlassen. So kann der erfindungsgemäße Reaktor selbstverständlich auch für andere katalytische Gasreaktionen eingesetzt werden. Weiterhin können die erfindungsgemäßen Reaktoren auch anders geschaltet werden, z.B. parallel oder in Reihe und parallel.

## Ansprüche

1. Reaktor (R) mit körniges Katalysatormaterial enthaltenden Rohren (4,17), die mit ihrem oberen Ende in einem oberen Rohrboden (5) und mit ihrem unteren Ende in einem unteren Rohrboden (6) angeordnet und in Betrieb von wenigstens den zwischen den Rohrböden gebildeten Raum durchströmenden Kühlmittel umgeben sowie von einem umzusetzenden Gasgemisch durchströmt sind, wobei einer der Rohrböden fest am Reaktormantel (7) angeordnet und der andere Rohrboden ohne unmittelbare Verbindung relativ zum Reaktormantel (7) bewegbar ausgebildet ist, dadurch gekennzeichnet, daß der bewegbare Rohrboden (5) mit einem eigenen Kugelboden bzw. Gasdom (3) ausgebildet und zwischen diesem Gasdom (3) und Reaktormantel (7) ein oder mehrere wenigstens bereichsweise flexible Kompensationsgasrohre (2) mit einer festen Verbindung zum Reaktormantel (7) vorgesehen ist bzw. sind.

2. Reaktor nach Anspruch 1 mit einem unteren festen Rohrboden, dadurch gekennzeichnet, daß der obere Rohrboden (5) gegenüber dem Reaktormantel (7,7c,7d) bewegbar ausgebildet und dessen Gasdom (3,3c,3d) mit dem oder den Kompensationsgasrohren (2,2c,2d) ausgerüstet ist (Fig. 1, Fig. 4, Fig. 5).

3. Reaktor nach Anspruch 1 mit einem unteren festen Rohrboden, dadurch gekennzeichnet, daß der obere Rohrboden (3a,3b) gegenüber dem Reaktormantel (7a,7b) bewegbar ausgebildet ist und das Kompensationsgasrohr (2a,2b) den Reaktor durchsetzt und mit seinem freien Ende unmittelbar mit dem bewegbaren oberen Rohrboden (3a,3b) verbunden ist (Fig. 2, Fig. 3).

4. Reaktor nach einem der vorangehenden Ansprüchen, dadurch gekennzeichnet, daß die dem Gasdom (3) zugeordneten Einströmbereiche des oder der Kompensationsrohre mit Gasverteilungseinrichtungen (27) ausgerüstet sind.

5. Reaktor nach einem der vorangehenden Ansprüche, dadurch gekennzeichnet, daß zwischen Außenfläche des Gasdomes (3) und der Innenfläche des Reaktormantels (7.12) ein Kühlmitteldampfdom (13) ausgebildet ist.

6. Reaktor nach Anspruch 5, dadurch gekennzeichnet, daß im Bereich des Kühlmitteldampfdomes (13) ein Kühlmitteldampfaustritt (22) mit Tröpfchenabscheider (14) angeordnet ist.

7. Reaktor nach einem der vorangehenden Ansprüche, dadurch gekennzeichnet, daß wenigstens am Austritt eines der körniges Katalysatormaterial enthaltenden Rohre (4) eine Gasdurchflußregeleinrichtung (18) vorgesehen ist.

8. Reaktor nach Anspruch 7, dadurch gekennzeichnet, daß das mit der Gasdurchflußregeleinrichtung (18) ausgerüstete Rohr (17) einen größeren Querschnitt als die übrigen Rohre (4) aufweist.

9. Verwendung eines Reaktors nach einem der Ansprüche 1 bis 8 zur Herstellung von Methanol aus einem Wasserstoff. Kohlenmonoxid und Kohlendioxid enthaltenden Synthesegas.

Fig.1

Fig.2

Fig.3

Fig.4

Fig.5

Fig.6

**Europäisches Patentamt**

# EUROPÄISCHER RECHERCHENBERICHT

Nummer der Anmeldung

# EP 90 12 0766

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 700 081   (STONE & WEBSTER ENGINEERING)<br>* Auszug; Seite 1, Zeilen 6-19; Seite 6, Zeilen 3-26; Seite 8, Zeile 18 - Seite 10, Zeile 9; Figur 1 *<br>– – – | 1,9 | B 01 J<br>8/06<br>C 07 C 29.15 |
| Y | | 2 | |
| X | FR-A-2 386 344   (PULLMAN)<br>* Seite 1, Zeile 33 - Seite 9, Zeile 4; Figur 1 *<br>– – – | 1 | |
| Y | | 2 | |
| A | US-A-4 224 298   (L.F. ROBINSON)<br>* Auszug; Figuren 2,3; Spalte 5, Zeilen 1-52 *<br>– – – – – | 1,7 | |

RECHERCHIERTE
SACHGEBIETE (Int. Cl.5)

B 01 J
C 01 B
C 07 C

Der vorliegende Recherchenbericht wurde für alle Patentansprüche erstellt

| Recherchenort | Abschlußdatum der Recherche | Prüfer |
|---|---|---|
| Den Haag | 26 November 90 | SIEM T.D. |